# EUROPÄISCHE PATENTANMELDUNG

(11) **EP 1 094 314 A1**
(43) Veröffentlichungstag der Anmeldung: **25.04.2001**
(21) Anmeldenummer: 00120048.4
(22) Anmeldetag: 15.09.2000
(51) Int. Cl.: G01N 31/12

(54) **Analysator zur Bestimmung der stofflichen Zusammensetzung von Materialproben**

(30) Priorität: 19.10.1999 DE 29918373 U
(71) Anmelder: ELTRA Entwicklungs- und Vetriebsgesellschaft von elektronischen und physikalischen Geräten mbH., 41469 Neuss (DE)
(72) Erfinder: Polemitis, Yiannakis, 41469 Neuss (DE)
(74) Vertreter: Paul, Dieter-Alfred, Dipl.-Ing.

(57) **Zusammenfassung**

Die Erfindung beschreibt einen Analysator zur Bestimmung der stofflichen Zusammensetzung von flüssigen oder festen Materialien mit einem Verbrennungsofen (1), um eine Materialprobe unter Zugabe eines Trägergases zu verbrennen, und einer mit dem Verbrennungsofen (1) verbundenen Meßeinrichtung (3) zur Analyse des bei der Verbrennung entstehenden Rauchgases, wobei der Verbrennungsofen (1) ein Verbrennungsrohr (20) aufweist, das an eine Trägergasleitung (2) angeschlossen und auslaßseitig mit der Meßeinrichtung (3) verbunden ist und das innenseitig einen Axialanschlag (23) aufweist, um in dem Verbrennungsrohr (20) einen Materialträger (24), auf dem die zu untersuchende Materialprobe angeordnet ist, zu positionieren, welche dadurch gekennzeichnet ist, daß das Verbrennungsrohr (20) im wesentlichen geradlinig ausgebildet und an seinem Ende an die Trägergasleitung (2) angeschlossen und an seinem anderen Ende mit der Meßeinrichtung (3) verbunden ist, und daß in das Verbrennungsrohr (20) ein Innenrohr (22) eingeschoben ist, das an seinem einlaßseitigen Endbereich den Axialanschlag (23) für den Materialträger (24) bildet und zudem einen über den Anschlag (25) zur Einlaßseite des Verbrennungsrohrs (20) vorstehenden Endabschnitt (25) aufweist, der derart ausgebildet ist, daß ein das Verbrennungsrohr (20) durchströmender Trägergasstrom durch den Endabschnitt (25) gegen den Materialträger (24) gelenkt wird.

## Beschreibung

Die vorliegende Erfindung betrifft einen Analysator zur Bestimmung der stofflichen Zusammensetzung von flüssigen oder festen Materialien mit einem Verbrennungsofen, um eine Materialprobe unter Zugabe eines Trägergases zu verbrennen, und einer mit dem Verbrennungsofen verbundenen Meßeinrichtung zur Analyse des bei der Verbrennung entstehenden Rauchgases, wobei der Verbrennungsofen ein Verbrennungsrohr aufweist, das an eine Trägergasleitung angeschlossen und auslaßseitig mit der Meßeinrichtung verbunden ist und das innenseitig einen Axialanschlag aufweist, um in dem Verbrennungsrohr einen Materialträger, auf dem die zu untersuchende Materialprobe angeordnet ist, zu positionieren.

Analysatoren dieser Art sind bekannt und werden eingesetzt, um insbesondere den Anteil von Kohlenstoff und Schwefel in Kohle, Öl, Asche, Katalysatoren, Kalk, Gips, Böden, Gummi, Abfall und anderen festen und flüssigen Proben schnell und simultan zu bestimmen. Die Messung der Schwefel- oder Kohlenstoffkonzentration wird auf dem Prinzip der Probenverbrennung und der Analyse der Verbrennungsgase mittels Infrarotabsorption durchgeführt. Hierzu wird eine Probe des zu untersuchenden Materials in Form von Pulver, Spänen, festen Stücken oder auch in flüssiger Form auf einem sogenannten Schiffchen liegend in ein Verbrennungsrohr eines Ofens eingeführt und verbrannt, wobei die in der Probe vorliegenden Schwefel- und Kohlenstoffanteile zu SO₂ und CO₂ oxidiert werden. Die üblichen Verbrennungstemperaturen liegen hier bei zwischen 1200 und 1450°C. Die Verbrennung erfolgt unter gleichzeitiger Zuführung von Sauerstoff, der auch als Trägergas für die bei der Verbrennung entstehenden Oxidationsprodukte SO₂ und CO₂ dient. Das Trägergas mit den Oxidationsprodukten wird dann über Staubfallen und Feuchtigkeitsabsorber einem nachgeschalteten Infrarotanalysator zugeführt. Die von dem Infrarotanalysator abgegebenen Signale sind selektiv und entsprechen der SO₂- bzw. CO₂-Konzentration im Gasgemisch. Sie werden von der Elektronik linearisiert, integriert, durch das Probengewicht, das zuvor ermittelt wurde, dividiert und in Prozent S und in Prozent C digital angezeigt.

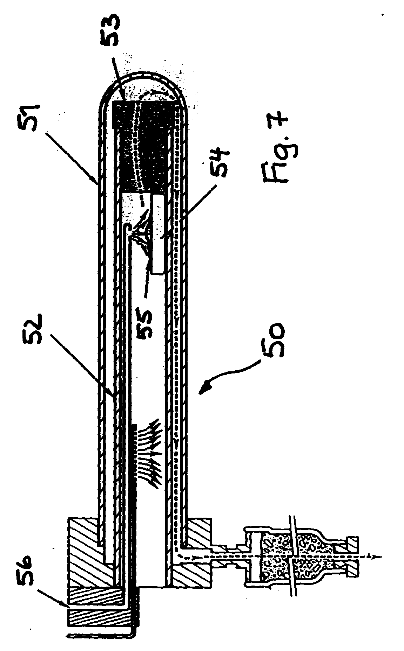

Bei einem bekannten Analysator, von dem die Erfindung ausgeht und der in der Figur 7 gezeigt ist, ist das Verbrennungsrohr 50 als Doppelrohr ausgebildet mit einem Außenrohr 51, das an seinem einen Ende geschlossen ist, und einem darin unter Bildung eines Ringraums gehaltenen Innenrohr 52, das an seinem zum geschlossenen Ende des Außenrohrs 51 weisenden Ende durch einen gasdurchlässigen Stopfen 53 verschlossen ist. Dieser Stopfen 53 bildet gleichzeitig einen Axialanschlag für einen die Probe 55 tragenden Materialträger 54, der auch Schiffchen genannt wird. Der für die Verbrennung erforderliche Sauerstoff wird der Probe über eine Lanze 56 zugeführt, die von dem offenen Ende des Verbrennungsrohrs 50 her in das Innenrohr 52 eingeführt ist. Die bei der Verbrennung entstehenden Oxidationsgase werden zusammen mit dem Sauerstoff durch den gasdurchlässigen Stopfen 53 und den zwischen dem Außenrohr 51 und dem Innenrohr 52 gebildeten Ringraum dem Infrarotanalysator zugeführt.

Das bekannte Verbrennungsrohr ist aufgrund seiner Ausbildung als Doppelrohranordnung mit einer hineinragenden Sauerstofflanze relativ kompliziert im Aufbau und auch anfällig gegen Beschädigungen.

Aufgabe der Erfindung ist es daher, einen Analysator der eingangs genannten Art so auszubilden, daß er wenig wartungs- und kostenintensiv ist.

Diese Aufgabe ist erfindungsgemäß dadurch gelöst, daß das Verbrennungsrohr im wesentlichen geradlinig ausgebildet ist und an seinem Ende an die Trägergasleitung angeschlossen und an seinem anderen Ende mit der Meßeinrichtung verbunden ist, und daß in das Verbrennungsrohr ein Innenrohr eingeschoben ist, das an seinem einlaßseitigen Endbereich den Axialanschlag für den Materialträger bildet und zudem einen über den Anschlag zur Einlaßseite des Verbrennungsrohrs vorstehenden Endabschnitt aufweist, der derart ausgebildet ist, daß ein Trägergasstrom durch den Endabschnitt gegen den Materialträger gelenkt wird.

Erfindungsgemäß ist somit zunächst das Verbrennungsrohr ein einfaches, geradliniges Röhr, das während der Verbrennung axial von dem Trägergas wie beispielsweise Sauerstoff durchströmt wird. Um hierbei zu gewährleisten, daß ausreichend Sauerstoff an die zu untersuchende Probe gelangt, sind besondere Vorkehrungen getroffen, indem in das Verbrennungsrohr von der Auslaßseite her ein Innenrohr eingeschoben ist, welches zum einen den Axialanschlag für den Materialträger bildet und zum anderen einen Endabschnitt aufweist, der so ausgebildet ist, daß er einen am Anschlag positionierten Materialträger teilweise überragt und dabei den zwischen dem Materialträger und der Innenrohrwandung verbleibenden Strömungskanal teilweise verschließt. Hierdurch wird erreicht, daß das Trägergas von dem Endabschnitt in Richtung des Materialträgers und damit auf die Probe geleitet wird, so daß ausreichend Sauerstoff für deren Verbrennung zur Verfügung steht. Das bei der Verbrennung mit entstehenden Oxidationgasen angereicherte Trägergas wird dann durch das Innenrohr weiter zur Meßeinrichtung geleitet.

Erfindungsgemäß besteht die Verbrennungsrohranordnung somit lediglich aus einem geradlinigen Verbrennungsrohr, in welches zusätzlich ein Innenrohr eingeschoben ist. Dies stellt eine deutliche Vereinfachung gegenüber der eingangs beschriebenen und in Fig. 7 dargestellten herkömmlichen Doppelrohranordnung mit einer zusätzlichen Sauerstofflanze dar. Ein weiterer Vorteil der erfindungsgemä-ßen Ausbildung besteht darin, daß durch das insbesondere aus Keramik bestehende Innenrohr verhindert wird, daß die heißen und zum Teil aggressiven Rauchgase an das Verbrennungsrohr gelangen und dieses beschädigen. Es kann allenfalls das Innenrohr Risse bekommen, die jedoch unschädlich sind, da Undichtigkeiten des Innenrohres nicht zu Meßgasverlusten führen.

Hinsichtlich weiterer vorteilhafter Ausgestaltungen der Erfindung wird auf die Unteransprüche sowie die nachfolgende Beschreibung eines Ausführungsbeispiels unter Bezugnahme auf die beiliegende Zeichnung verwiesen. In der Zeichnung zeigt:
- Figur 1: eine Prinzipschaltskizze eines Analysators gemäß der vorliegenden Erfindung,
- Figur 2: den Verbrennungsofen des in Figur 1 dargestellten Analysators,
- Figur 3: in Unteransicht ein Innenrohr zur Verwendung in dem Verbrennungsofen gemäß Figur 2,
- Figur 4: das Innenrohr aus Figur 3 in Seitenansicht,
- Figur 5: das Innenrohr aus Figur 3 in Frontansicht,
- Figur 6: die Einzelheit X aus Figur 2 in vergrößerter Darstellung und
- Figur 7: eine Verbrennungsrohranordnung gemäß dem Stand der Technik.

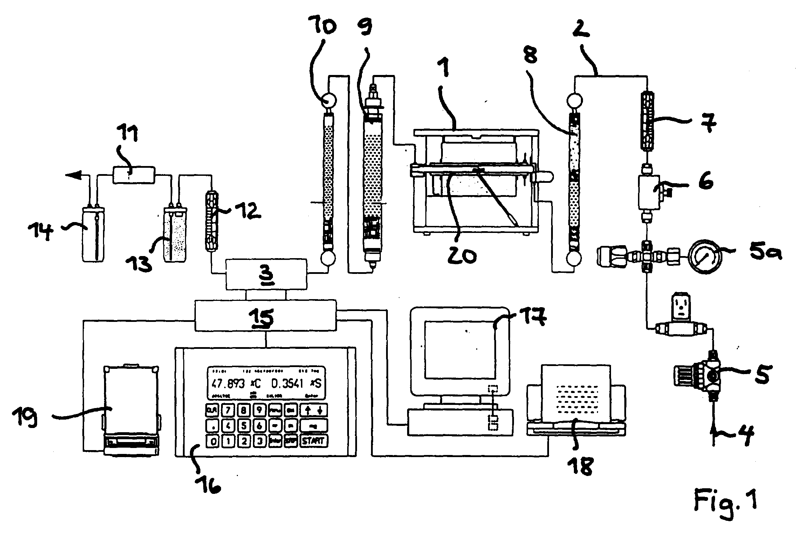

In Figur 1 ist eine Prinzipskizze eines Analysators gemäß der vorliegenden Erfindung dargestellt, mit dem Materialien wie beispielsweise Kohle, Asche, Öl usw. auf ihre Bestandteile, insbesondere auf ihre Schwefel- und Kohlenstoffanteile, untersucht werden. Der Analysator arbeitet auf dem Prinzip der Probenverbrennung und der Analyse der Verbrennungsgase mittels Infrarotabsorption und umfaßt als zentrales Bauelement einen Verbrennungsofen 1, in dem die zu untersuchende Materialprobe verbrannt wird und der einlaßseitig an eine Trägergasleitung 2, über die dem Verbrennungsofen 1 Sauerstoff zugeführt wird, und auslaßseitig an eine Infrarotzelle 3, in der das Trägergas und die darin enthaltenen Oxidationsgase, die bei der Verbrennung der Materialprobe entstanden sind, zu analysieren, angeschlossen ist. Die Trägergasleitung 2 ist an eine Sauerstoffquelle 4 angeschlossen, über die dem System handelsüblicher, technisch reiner Sauerstoff, wie er beispielsweise in Stahlflaschen lieferbar ist, mit einer Reinheit von 99,5% zugeführt wird. In die Trägergasleitung 2 ist weiterhin eingebaut ein Druckregler 5, der dafür sorgt, daß der Sauerstoffdruck ca. 1,5 bar beträgt. Der Druck wird an einem Manometer 5a angezeigt.

Dem Manometer 5 nachgeschaltet ist eine Drossel 6, über die ein gewünschter Durchfluß von ca. 200 l/h eingestellt werden kann, der an einem Durchflußmesser 7 abgelesen werden kann. Zwischen Durchflußmesser 7 und Verbrennungsofen 1 ist eine H₂O-Falle 8 geschaltet, in der eventuell im Sauerstoffstrom enthaltene Feuchtigkeit festgehalten wird. Bei Geräten, die Kohlenstoff im ppm-Bereich bestimmen, z.B. bei der Stahlanalyse, kann die erste Hälfte dieser Falle mit CO₂-Absorptionsmittel, und die zweite Hälfte mit Feuchtigkeitsabsorptionsmittel gefüllt werden. Als H₂O-Absorptionsmittel kann beispielsweise Magnesiumperchlorat (Anhydrone) dienen, und als CO₂-Absorptionsmittel wird in bevorzugter Weise Natrium-hydroxid (Askarite), am besten mit Indikator, durch dessen Verfärbung der gesättigte Zustand sichtbar gemacht wird, eingesetzt.

Zwischen dem Verbrennungsofen 1 und der Infrarotzelle 3 sind zwei H₂O-Fallen 9, 10 vorgesehen, da die aus dem Verbrennungsofen 1 austretenden Gase in der Regel Feuchtigkeit enthalten. Dies ist insbesondere bei der Analyse von Kohleproben der Fall, weil die Proben oft nicht ganz trocken sind und die Proben auch Wasserstoff enthalten, die bei der Verbrennung der Probe mit dem Verbrennungssauerstoff zu H₂O reagieren.

Der Infrarot-Zelle 3 ist eine Pumpe 11 nachgeschaltet, durch die der Durchfluß des Systems erzeugt wird. Die Pumpe 11 wird durch eine Elektronik geregelt und der Durchfluß von einem Meßgasdurchflußsensor 12 gemessen und angezeigt. Der Pumpe 11 vor- und nachgeschaltet sind Dämpfungsvolumina 13, 14, um ein Pulsieren des Meßgases durch die Membranschwingungen der Pumpe 11 zu verhindern.

An die Infrarot-Zelle 3 angeschlossen ist ferner eine Elektronik 15, an die eine Tastatur mit einer Anzeige 16, ein Monitor 17, ein Drucker 18 sowie eine Waage 19 angeschlossen sind.

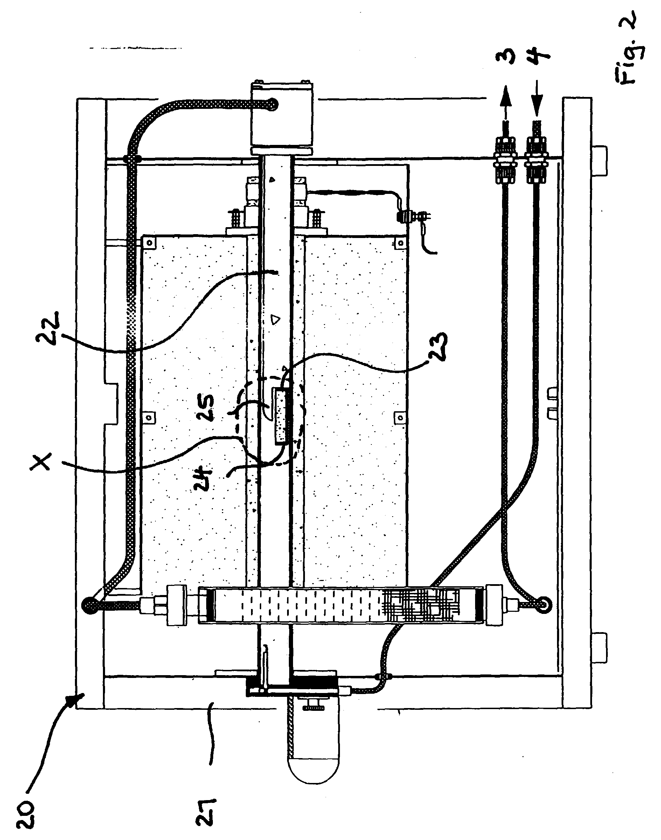

In dem Verbrennungsofen 1, der im einzelnen in Figur 2 dargestellt ist, läuft die Verbrennung in einem Verbrennungsrohr 20 ab, welche sich zwischen den beiden Stirnseiten der ofenwandung 21 geradlinig erstreckt und an seinem einen einlaßseitigen axialen Ende an die von der Sauerstoffquelle 4 kommende Trägergasleitung 2 und an seinem anderen, auslaßseitigen Ende an den zur Infrarot-Zelle 3 führenden Teil der Gasleitung angeschlossen ist. In dem Verbrennungsrohr 20 ist ein Innenrohr 22 vorgesehen, das in das Verbrennungsrohr 20 von dessen Auslaßseite her eingeschoben ist und an seinem zur Einlaßseite des Verbrennungsrohrs 20 weisenden Endbereich einen Axialanschlag 23 für einen Materialträger 24, auch Schiffchen genannt, auf dem die zu untersuchende Probe vorgesehen ist, bildet.

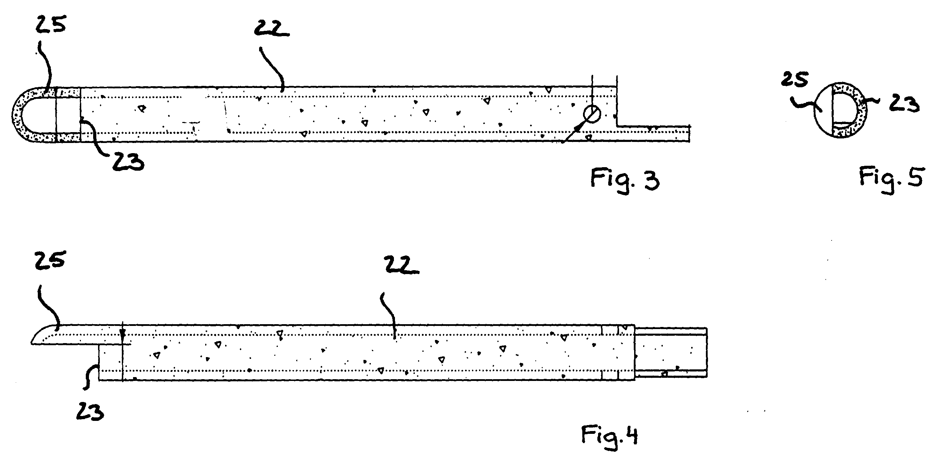

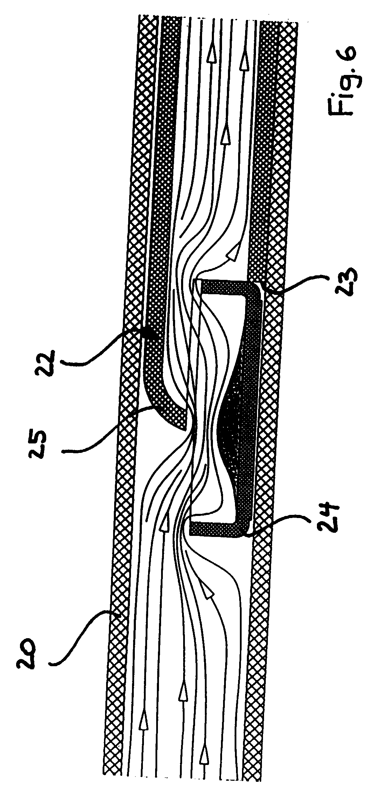

Wie im einzelnen in den Figuren 3 bis 5 gezeigt ist, besitzt das Innenrohr 22 einen über den Axialanschlag 23 vorstehenden Endabschnitt 25, der so ausgebildet ist, daß er das an dem Anschlag 23 positionierte Schiffchen 24 bis etwa zu dessen Mitte übergreift und dabei den zwischen der Schiffchenoberseite und der Rohrinnenwandung gebildeten Freiraum im wesentlichen verschließt. Die Spitze des Endabschnitts 25 ist dabei nach Art einer Nase in Richtung des Schiffchens 24 heruntergezogen, so daß ein durch das Verbrennungsrohr 20 strömendes Gas durch den Endabschnitt 25 in das Schiffchen 24 gelenkt wird und durch dieses hindurch in das Innenrohr 22 einströmt. Dieses Strömungsverhalten ist in Figur 6 schematisch dargestellt. Im übrigen lassen die Figuren 3 bis 5 erkennen, daß das Innenrohr 22 aus einem reagenzglasartig ausgebildeten Rohr hergestellt ist, das an seinem geschlossenen Endbereich unter Bildung des Axialanschlags 23 sowie des vorstehenden Endabschnitts 25 über einen Umfangsbereich von insbesondere 180°-210° aufgeschnitten ist.

Der Verbrennungsofen 1 ist widerstandsgeheizt. Als Heizelemente dienen Siliziumkarbidstäbe. Im Gegensatz zu Öfen mit Heizdrahtwicklung können auf diese Weise Dauertemperaturen von weit über 1000°C erreicht werden.

Der Verbrennungsofen 1 ist voll elektronisch gesteuert und daher transformatorlos. Die Elektronik regelt die Temperatur und begrenzt zugleich den Maximaistrom während der Aufheizphase. Der Sollwert der Temperatur wird manuell durch einen Drehknopf am Ofen eingestellt und von einer am Ofen eingebauten Digitalanzeige mit einer Auflösung von 1°C angezeigt.

Der Ist-Wert von Temperatur wird von einem Thermoelement geliefert und über eine Regelung dem Soll-Wert angeglichen.

Ein weiterer Temperaturfühler erfaßt die Umgebungstemperatur und korrigiert den Referenzpunkt des Thermoelements, so daß Umgebungstemperaturschwankungen keinen Einfluß auf die Ofentemperatur haben.

Die Funktionsweise des erfindungsgemäßen Analysators ist wie folgt:

Zunächst wird die zu untersuchende Materialprobe in Form eines Pulvers, von Spänen, festen Stücken oder auch in flüssiger Form auf dem Schiffchen 24 positioniert, und das Schiffchen 24 wird dann in das Verbrennungsrohr 20 durch eine Proben-Einführöffnung eingeschoben, bis es an dem Anschlag 23 des Innenrohrs 22 anliegt. Die Verbrennung erfolgt dann unter Zufuhr von Sauerstoff, der von der Sauerstoffquelle 4 mit einem Druck von 1,5 bar, der über den Druckregler 5 am System eingestellt werden kann, zugeführt. Mit Hilfe der einstellbaren Drossel 6 wird dabei ein Durchfluß von ca. 200 l/h eingestellt. Der Wert wird mit dem unteren Durchflußmesser 7 auf der Frontseite abgelesen. Diese Durchflußmenge wird dem Verbrennungsofen 1 über die H₂O-Falle 8 zugeführt, wobei der zugehörige Schlauchanschluß sich unterhalb des Schiffchen-Ablage des Ofeneingangs befindet.

Durch die im Ofen 1 herrschenden Temperaturen wird die Materialprobe im Schiffchen 24 unter der Zufuhr des Sauerstoffs, der gleichzeitig als Trägergas dient, verbrannt. Mit Hilfe des über das Schiffchen 24 ragenden Endabschnitt 25 des Innenrohrs 22 wird dabei der Sauerstoffstrom, wie in Figur 6 dargestellt, in das Schiffchen 24 gelenkt, so daß hier für die Verbrennung ausreichend Sauerstoff zur Verfügung steht. Die Verbrennungsgase werden dann mit dem Sauerstoff durch das Innenrohr 22 zum Ofenausgang durch den Unterdruck, welcher durch die Pumpe 11 erzeugt wird, abgesaugt, und zwar mit einer Durchflußmenge von 180 l/h. Diese Durchflußmenge wird konstant gehalten und ist geringer als der Durchfluß, der am Ofeneingang ankommt. Der Überschuß strömt aus dem Verbrennungsrohr 1 durch die Proben-Einführöffnung ins Freie, wodurch gewährleistet wird, daß keine Atmosphärenluft in den Verbrennungsraum eindringt.

Der aus dem Verbrennungsofen 1 austretende Gasstrom wird in den beiden nachgeschalteten H₂O-Fallen 9, 10 von Feuchtigkeit befreit, damit sie für die anschließende Analyse in der Infrarot-Zelle 3 ausreichend trocken sind. Schließlich wird das Gas durch den oberen Meßgasdurchflußmesser 12 auf der Frontseite des Analysengeräts geführt. Der angezeigte Wert muß konstant ca. 180 l/h betragen. Der Durchfluß wird von der Pumpe 11 erzeugt, die durch eine Elektronik geregelt wird. Ein Pulsieren des Meßgases durch die Membranschwingungen der Pumpe 11 wird durch die Dämpfungsvolumina 13, 14 vor und hinter der Pumpe 11 verhindert.

## Patentansprüche

1. Analysator zur Bestimmung der stofflichen Zusammensetzung von flüssigen oder festen Materialien mit einem Verbrennungsofen (1), um eine Materialprobe unter Zugabe eines Trägergases zu verbrennen, und einer mit dem Verbrennungsofen (1) verbundenen Meßeinrichtung (3) zur Analyse des bei der Verbrennung entstehenden Rauchgases, wobei der Verbrennungsofen (1) ein Verbrennungsrohr (20) aufweist, das an eine Trägergasleitung (2) angeschlossen und auslaßseitig mit der Meßeinrichtung (3) verbunden ist und das innenseitig einen Axialanschlag (23) aufweist, um in dem Verbrennungsrohr (20) einen Materialträger (24), auf dem die zu untersuchende Materialprobe angeordnet ist, zu positionieren, **dadurch gekennzeichnet,** daß das Verbrennungsrohr (20) im wesentlichen geradlinig ausgebildet und an seinem Ende an die Trägergasleitung (2) angeschlossen und an seinem anderen Ende mit der Meßeinrichtung (3) verbunden ist, und daß in das Verbrennungsrohr (20) ein Innenrohr (22) eingeschoben ist, das an seinem einlaßseitigen Endbereich den Axialanschlag (23) für den Materialträger (24) bildet und zudem einen über den Anschlag (25) zur Einlaßseite des Verbrennungsrohrs (20) vorstehenden Endabschnitt (25) aufweist, der derart ausgebildet ist, daß ein das Verbrennungsrohr (20) durchströmender Trägergasstrom durch den Endabschnitt (25) gegen den Materialträger (24) gelenkt wird.

2. Analysator nach Anspruch 1, **dadurch gekennzeichnet,** daß das Innenrohr (22) aus Keramik besteht.

3. Analysator nach einem der vorherigen Ansprüche, **dadurch gekennzeichnet,** daß der über den Axialanschlag (23) vorstehende Endabschnitt (25) des Innenrohrs (22) an seinem freien Ende so ausgebildet ist, daß er den Raum zwischen der Oberkante des Materialträgers (24) und der Innenwandung des Verbrennungsrohrs (20) im wesentlichen schließt, so daß ein das Verbrennungsrohr (20) durchströmendes Trägergas im wesentlichen nur unter Durchströmung des Materialträgers (25) in das Innenrohr (22) gelangen kann.

4. Analysator nach Anspruch 3, **dadurch gekennzeichnet,** daß das Innenrohr (22) aus einem reagenzglasartig ausgebildeten Rohr hergestellt ist, das an seinem geschlossenen Endbereich unter Bildung des Axialanschlags (23) sowie des vorstehenden Endabschnitts (25) über einen Umfangsbereich von insbesondere 180°-210° aufgeschnitten ist.
